(19) Europäisches Patentamt
European Patent Office
Office européen des brevets

(11) **EP 3 601 220 B1**

(12) **FASCICULE DE BREVET EUROPEEN**

(45) Date de publication et mention de la délivrance du brevet:
**05.05.2021  Bulletin 2021/18**

(21) Numéro de dépôt: **18714331.8**

(22) Date de dépôt: **19.03.2018**

(51) Int Cl.:
***C07C 309/15*** ^(2006.01)

(86) Numéro de dépôt international:
**PCT/FR2018/050659**

(87) Numéro de publication internationale:
**WO 2018/172682 (27.09.2018 Gazette 2018/39)**

(54) **PROCEDE DE RECUPERATION ASSISTEE DU PETROLE UTILISANT UN (CO)POLYMERE D'UNE FORME CRISTALLINE HYDRATEE DE L'ACIDE 2-ACRYLAMIDO-2-METHYLPROPANE SULFONIQUE**

VERBESSERTES ÖLRÜCKGEWINNUNGSVERFAHREN UNTER VERWENDUNG EINER HYDRIERTEN KRISTALLINEN FORM VON 2-ACRYLAMIDO-2-METHYLPROPANSULFONSÄURE

ENHANCED OIL RECOVERY METHOD USING A (CO)POLYMER OF A HYDRATED CRYSTALLINE FORM OF 2-ACRYLAMIDO-2-METHYLPROPANE SULFONIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **20.03.2017  FR 1752288**

(43) Date de publication de la demande:
**05.02.2020  Bulletin 2020/06**

(73) Titulaire: **SPCM SA**
**42160 Andrézieux-Bouthéon (FR)**

(72) Inventeurs:
• **FAVERO, Cédrick**
**42160 Andrezieux Boutheon (FR)**
• **KIEFFER, Johann**
**42160 Andrezieux Boutheon (FR)**
• **DAGUERRE, Frédéric**
**42160 Andrezieux Boutheon (FR)**

(74) Mandataire: **Cabinet Laurent & Charras**
**Le Contemporain**
**50 Chemin de la Bruyère**
**69574 Dardilly Cedex (FR)**

(56) Documents cités:
**FR-A1- 2 940 348    US-A1- 2010 274 048**

**Description**

DOMAINE DE L'INVENTION

La présente invention concerne un procédé de récupération assistée du pétrole et du gaz utilisant des (co)polymères hydrosolubles fabriqués à partir de la forme cristalline hydratée de l'acide 2-acrylamido-2-méthylpropane sulfonique (ATBS) ou d'au moins un de ses sels.

ETAT ANTERIEUR DE LA TECHNIQUE

La plupart des champs de pétrole actuellement exploités sont devenus matures et ont, de fait, amorcé le déclin de leur production ou sont sur le point de le faire. Le taux de récupération de ces champs est actuellement de l'ordre de 15 à 35 % en moyenne par rapport à la quantité initiale de pétrole. Ils offrent donc un potentiel de production encore considérable.

De manière générale, la récupération du pétrole brut contenu dans les gisements s'effectue en plusieurs temps.

La production résulte d'abord de l'énergie naturelle des fluides et de la roche qui se décompriment. A l'issue de cette phase de déplétion, la quantité de pétrole récupérée en surface représente en moyenne quelques 5 à 15 % de la réserve initiale. Il est donc nécessaire, dans un deuxième temps, d'employer des techniques visant à accroître le rendement de récupération en maintenant la pression du champ.

La méthode la plus fréquemment mise en œuvre consiste à injecter de l'eau dans le gisement par le biais de puits injecteurs dédiés à cette fin. On parle alors de récupération secondaire. Cette deuxième phase s'arrête lorsque le rapport eau/pétrole est trop important, c'est-à-dire lorsque la quantité en eau dans le mélange produit par les puits producteurs est trop importante. Cette récupération secondaire permet ainsi d'obtenir un taux de récupération additionnel de l'ordre de 10 à 20 %.

Les autres techniques utilisables sont regroupées sous le nom de récupération assistée du pétrole (RAP ou EOR acronyme anglais de « Enhanced Oil Recovery »). Leur but est de récupérer entre 10 et 35 % de pétrole additionnel par rapport à la quantité initiale de pétrole. Sous le terme de récupération assistée du pétrole, sont connues diverses techniques thermiques, ou non, telles que les techniques dites électrique, miscible, vapeur, ou encore chimique de récupération améliorée du pétrole restant en place (voir « Oil & gas science and technology » - revue IFP, vol 63 (2008) n°1, pp 9-19).

Par « pétrole », on désigne tout type d'huile, à savoir huile légère comme huile lourde, voire bitumineuse. Une huile résulte généralement de la transformation naturelle de matière organique et est composée d'un mélange d'hydrocarbures. Dans la description de l'art antérieur ou de l'invention, les termes « pétrole » et « huile » sont utilisés pour désigner la même matière, à l'exception de la mention de la composition d'une émulsion ou d'une dispersion.

L'efficacité du balayage par injection d'eau est généralement améliorée par l'addition de (co)polymères hydrosolubles. Les bénéfices attendus et prouvés de l'utilisation de (co)polymères, au travers de la « viscosification » des eaux injectées, sont l'amélioration du balayage et la réduction du contraste de viscosité entre les fluides pour maîtriser leur ratio de mobilité dans le champ, et ce afin de récupérer le pétrole rapidement et efficacement. Ces (co)polymères augmentent la viscosité de l'eau.

Il est connu de l'homme du métier que les (co)polymères hydrosolubles synthétiques, en particulier les (co)polymères à base d'ATBS, sont des (co)polymères très avantageux pour augmenter la viscosité des solutions aqueuses et sont utilisés en récupération assistée. En effet les (co)polymères à base d'ATBS sont connues pour être tolérants aux sels divalents ainsi qu'aux hautes températures.

En plus d'augmenter la viscosité de l'eau les polymères utilisés doivent avoir une bonne filtrabilité. Les (co)polymères possédant une mauvaise filtrabilité ont tendance à boucher la formation et ralentir voire inhiber la production de pétrole. Or, la filtrabilité se détériore lorsque le poids moléculaire du (co)polymère augmente. Il existe donc un compromis délicat entre poids moléculaire et filtrabilité.

Les (co)polymères ajoutés à l'eau d'injection sont généralement soumis à de longs temps de séjour dans le gisement, entre les puits injecteurs et les puits producteurs, pouvant aller de quelques mois à quelques années. Durant ce laps de temps, ils peuvent subir une dégradation thermique se traduisant par une augmentation de leur taux d'hydrolyse par conversion d'unités acrylamide ou ATBS en acrylates, ou une dégradation chimique conduisant à une rupture de chaine (baisse du poids moléculaire) par attaque radicalaire. Dans les deux cas, ces mécanismes se traduisent généralement par une diminution de la viscosité et donc par une perte de l'efficacité de balayage du pétrole par la solution aqueuse de polymère injectée dans le réservoir. Il y a donc un réel intérêt à développer des polymères plus résistants à ces processus impliqués dans tout projet de récupération assistée du pétrole ou du gaz FR2940348 porte sur la récupération assistée du pétrole par polymères préparés à partir de monomères comme l'acide 2-acrylamido-2-méthylpropane sulfonique.

Un objet de la présente invention est de fournir des polymères hydrosolubles qui présentent des propriétés améliorées notamment en termes de filtrabilité et de stabilité chimique et thermique améliorée, particulièrement utiles dans les techniques de récupération assistée du pétrole ou du gaz.

EXPOSE DE L'INVENTION

L'invention concerne l'utilisation d'une nouvelle forme cristalline hydratée de l'acide 2-acrylamido-2-méthylpropane sulfonique pour la préparation de (co)polymères hydrosolubles.

Par « (co)polymère hydrosoluble », on désigne un (co)polymère pouvant être solubilisé dans l'eau ou dans une saumure dans les conditions conventionnelles rencontrées dans les procédés de récupération assistée du pétrole ou du gaz, généralement entre 10 ppm et 15 000 ppm en poids comme indiqué ci-après.

D'autre part, et sauf contre-indication, par « acide 2-acrylamido-2-méthylpropane sulfonique sous forme cristalline hydratée » on désigne la forme acide et/ou la forme salifiée. Il en est de même pour les monomères anioniques mentionnés ci-après qui peuvent désigner les formes acides et/ou salifiées comme, par exemple, pour l'acide acrylique.

La forme saline est avantageusement obtenue à partir d'un composé choisi parmi un hydroxyde de métal alcalin ou alcalino-terreux, un oxyde de métal alcalin ou alcalino-terreux, l'ammoniaque, une amine de formule suivante $NR_1R_2R_3$ ($R_1$, $R_2$ et $R_3$ étant avantageusement des groupements hydrocarbonés, notamment des alkyles) ou un carbonate de métal alcalin ou alcalino-terreux. Un métal alcalin préféré est le sodium.

La forme acide d'un monomère peut être salifiée avant et/ou pendant et/ou après la (co)polymérisation du ou des monomères.

Un autre aspect de l'invention concerne un procédé de récupération assistée du pétrole caractérisé en ce qu'il comprend les étapes suivantes :

    a) Préparation d'un fluide d'injection comprenant au moins un (co)polymère hydrosoluble fabriqué à partir de la forme cristalline hydratée de l'acide 2-acrylamido-2-méthylpropane sulfonique ou d'au moins un de ses sels, avec de l'eau ou de la saumure,
    b) Injection du fluide d'injection dans une formation souterraine,
    c) Balayage de la formation souterraine à l'aide du fluide injecté,
    d) Récupération d'un mélange aqueux et hydrocarboné.

La forme cristalline hydratée de l'acide 2-acrylamido-2-méthylpropane sulfonique a un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta. L'incertitude de ces pics est généralement de l'ordre de 0.1°.

La cristallographie aux rayons X, radiocristallographie ou diffractométrie de rayons X est une technique d'analyse qui permet d'étudier la structure de la matière cristalline à l'échelle atomique. Elle s'appuie sur le phénomène physique de diffraction des rayons X. Un diffractomètre ayant une source au cuivre peut être utilisé.

Une poudre formée d'une phase cristalline particulière va toujours donner lieu à des pics de diffraction dans les mêmes directions. Ce diagramme de diffraction forme ainsi une véritable signature de la phase cristalline. Il est donc possible de déterminer la nature de chaque phase cristalline au sein d'un mélange ou d'un produit pur.

Cette signature est spécifique à chaque composé cristallin organique ou inorganique, et se présente sous la forme d'une liste de pics de position en angle $2\theta$ (2-thêta).

Cette technique est utilisée pour caractériser la matière, en particulier les différentes formes cristallines qui peuvent exister pour une même molécule chimique.

La forme cristalline hydratée de l'acide 2-acrylamido-2-méthylpropane sulfonique a un spectre infra-rouge à transformée de Fourier comprenant des pics à $3280 cm^{-1}$, $3126 cm^{-1}$, $1657 cm^{-1}$, $1595 cm^{-1}$, $1453 cm^{-1}$, $1395 cm^{-1}$, $1307 cm^{-1}$, $1205 cm^{-1}$, $1164 cm^{-1}$, $1113 cm^{-1}$, $1041 cm^{-1}$, $968 cm^{-1}$, $885 cm^{-1}$, $815 cm^{-1}$, $794 cm^{-1}$. L'incertitude de ces pics est généralement de l'ordre de $8 cm^{-1}$. De manière avantageuse, il s'agit du spectre solide obtenu conventionnellement dans un sel tel que le KBr.

La spectroscopie infra-rouge à transformée de Fourier est l'analyse des vibrations émises, absorbées ou diffusées par les molécules. Cette technique est sensible aux interactions dites courtes (influence de la maille unitaire sur les liaisons). Dans la majorité des cas, les spectres infra-rouges à transformée de Fourier de différents systèmes cristallins diffèrent de manière significative. Le spectre infra-rouge à transformée de Fourier reflète donc les détails de la structure cristalline d'un composé organique.

De manière générale, et sauf indication contraire, le diagramme de diffraction des rayons X et le spectre infra-rouge sont obtenus à 20°C et à une pression de 1 atmosphère (101 325 Pa).

La forme cristalline hydratée de l'acide 2-acrylamido-2-méthylpropane sulfonique a une énergie minimale d'inflammation supérieure à 400 mJ, préférentiellement supérieure à 500 mJ (1 mJ = $10^{-3}$ joule).

L'énergie minimale d'inflammation représente l'énergie minimale qui doit être fourni à un composé pour provoquer une inflammation. L'énergie peut être électrique ou thermique. L'énergie minimale d'inflammation est une donnée essentielle pour la prise en compte du risque d'explosion lors de la manipulation du produit (transfert, stockage, réaction, mise en forme...).

L'énergie minimale d'inflammation dépend des propriétés de la poudre (composition) ainsi que de sa structure macromoléculaire (granulométrie, forme cristalline, surface spécifique).

Dans le cadre des solides, cette énergie est l'énergie minimale d'une étincelle électrique susceptible d'enflammer un nuage de poussière. Plus la valeur de l'énergie minimale d'inflammation est élevée, moins le solide présente un risque lors de son utilisation, manipulation, stockage.

La mesure de l'énergie minimale d'inflammation est réalisée selon la norme NF EN 13821.

La forme cristalline hydratée de l'acide 2-acrylamido-2-méthylpropane sulfonique présente 4 phénomènes thermiques avec la technique de calorimétrie différentielle à balayage, à 70°C, 100°C, 150°C et 190°C. L'incertitude relative à l'observation de ces phénomènes est généralement de l'ordre de 10°C, avantageusement 5°C ou moins.

Les phénomènes thermiques sont mesurés par calorimétrie différentielle à balayage (DSC). Cette technique exploite la mesure de la variation de chaleur associée à la dénaturation thermique du composé lorsque celui-ci est chauffé à vitesse constante, par exemple avec une rampe de chauffe de 10°C/minute.

Il est généralement reconnu que le phénomène thermique se présentant à 190°C (+/-10°C) est lié au point de fusion de l'acide 2-acrylamido-2-méthylpropane sulfonique.

L'invention a pour objet l'utilisation d'une nouvelle forme cristalline hydratée de l'acide 2-acrylamido-2-méthylpropane sulfonique ou d'au moins un de ses sels pour la préparation de (co)polymère hydrosoluble.

Selon un autre mode particulier de l'invention, le (co)polymère hydrosoluble est obtenu au moins à partir d'acide 2-acrylamido-2-méthylpropane sulfonique dont 50% à 100% est sous la forme cristalline hydratée, plus avantageusement 70 à 100%, et encore plus avantageusement 100%.

Le (co)polymère hydrosoluble est avantageusement obtenu à partir d'entre 1 et 100 mol% d'acide 2-acrylamido-2-méthylpropane sulfonique, préférentiellement entre 5 et 100 mol% d'acide 2-acrylamido-2-méthylpropane sulfonique, encore plus préférentiellement entre 25 et 100 mol% d'acide 2-acrylamido-2-méthylpropane sulfonique ; 50% à 100 % de l'acide 2-acrylamido-2-méthylpropane sulfonique étant avantageusement sous la forme cristalline hydratée, plus avantageusement 70 à 100%, et encore plus avantageusement 100%.

De manière générale, l'homme du métier saura, le cas échéant, ajuster la quantité des éventuels monomères additionnels (anionique et/ou cationique et/ou zwitterionique) listés ci-après pour atteindre 100mol%.

Selon un autre mode particulier de l'invention le (co)polymère hydrosoluble est obtenu à partir d'acide 2-acrylamido-2-méthylpropane sulfonique dont 50% à 100% est avantageusement sous la forme cristalline hydratée (plus avantageusement 70 à 100%, et encore plus avantageusement 100%) et d'au moins un monomère non ionique et/ou au moins un monomère anionique et/ou au moins un monomère cationique et/ou un monomère zwitterionique.

Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide, le N,N-diéthylacrylamide et le N-méthylolacrylamide. Egalement, peuvent être utilisés la N-vinylformamide, le N-vinyl acétamide, la N-vinylpyridine et la N-vinylpyrrolidone, le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprénol. Un monomère non ionique préféré est l'acrylamide.

Selon un mode de réalisation particulier, le (co)polymère est avantageusement obtenu à partir d'entre 1 et 99,9 mol% de monomère(s) non-ionique(s), de préférence entre 40 et 95 mol% et plus préférentiellement entre 45 et 90 mol%, par rapport au nombre total de monomères. Dans ce cas, le copolymère est avantageusement obtenu à partir d'entre 0,1 et 99 mol% d'acide 2-acrylamido-2-méthylpropane sulfonique ; 50% à 100% de l'acide 2-acrylamido-2-méthylpropane sulfonique étant avantageusement sous la forme cristalline hydratée, plus avantageusement 70 à 100%, et encore plus avantageusement 100%.

Le ou les monomères anioniques pouvant être utilisés dans le cadre de l'invention peuvent être choisis dans un large groupe. Ces monomères peuvent présenter des fonctions acryliques, vinyliques, maléiques, fumariques, malonique, itaconique, allyliques et contenir un groupe carboxylate, phosphonate, phosphate, sulfate, sulfonate, ou un autre groupe à charge anionique. Le monomère anionique peut être sous forme acide ou bien sous forme de sel de métal alcalino-terreux, de sel de métal alcalin ou de sel d'ammonium. Des exemples de monomères incluent l'acide acrylique ; l'acide méthacrylique ; l'acide itaconique ; l'acide crotonique ; l'acide maléique ; l'acide fumarique, l'acide acrylamido undécanoïque, l'acide 3-acrylamido 3-méthylbutanoïque, l'anhydride maléique ; les monomères de type acide fort présentant par exemple une fonction de type acide sulfonique ou acide phosphonique tels que l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylmethacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium. Dans cette liste, les monomères mentionnés de type acide fort présentant une fonction de type acide sulfonique n'incluent pas la forme cristalline hydratée de l'acide 2-acrylamido-2-méthylpropane sulfonique.

Selon un mode de réalisation particulier, le copolymère est avantageusement obtenu à partir d'entre 1 et 99 mol% de monomère(s) anionique(s), de préférence entre 5 et 60 mol%, et encore plus préférentiellement entre 10 et 50 mol%, par rapport au nombre total de monomères. Dans ce cas, ces pourcentages incluent également le monomère de forme cristalline hydratée de l'acide 2-acrylamido-2-méthylpropane sulfonique selon l'invention.

Le ou les monomères cationiques pouvant être utilisés dans le cadre de l'invention peuvent être choisis, notamment parmi les monomères dérivés de motifs de type acrylamide, acrylique, vinylique, allylique ou maléique, ces monomères possédant une fonction phosphonium ou ammonium quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de methacrylamido propyltrimethyl ammonium (MAPTAC). L'agent de quaternisation peut être choisi parmi les chlorures d'alkyle, les sulfates de dialkyle ou les halogénures d'alkyle. Préférentiellement l'agent de quaternisation est choisi parmi le chlorure de méthyle ou le diéthyle sulfate.

Le monomère zwitterionique peut être un dérivé d'un motif de type acrylamide, acrylique, vinylique, allylique ou maléique possédant une fonction amine ou ammonium quaternaire et une fonction acide de type carboxylique (ou carboxylate), sulfonique (ou sulfonate) ou phosphorique (ou phosphate). On peut citer, en particulier et de façon non limitative les dérivés de l'acrylate de diméthylaminoéthyl, tel que le 2-((2-(acryloyloxy)éthyl) diméthylammonio) éthane-1-sulfonate, le 3-((2-(acryloyloxy)éthyl)diméthylammonio) propane-1-sulfonate, le 4-((2-(acryloyloxy)éthyl) diméthylammonio) butane-1-sulfonate, le [2-(acryloyloxy)éthyl] (diméthylammonio) acetate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl]] (diméthylammonio) acétate, les dérivés du diméthylamino propylacrylamide tel que le 2-((3-acrylamidopropyl)diméthylammonio) éthane-1-sulfonate, le 3-((3-acrylamidopropyl)diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

Des monomères présentant un caractère hydrophobe peuvent également être utilisés dans l'invention. Ils sont de préférence choisis dans le groupe constitué par les esters de l'acide (méth)acrylique présentant une chaîne alkyle, arylalkyle, propoxylée, éthoxylée, ou éthoxylée et propoxylée ; les dérivés du (méth)acrylamide présentant une chaîne alkyle, arylalkyle propoxylée, éthoxylée, éthoxylée et propoxylée, ou dialkyle ; les alkyl aryl sulfonates.

Lorsqu'on utilise un monomère présentant un caractère hydrophobe, sa quantité se situe avantageusement dans la plage comprise entre 0,001 et 3 % en mole par rapport à la quantité totale de monomères.

Les monomères présentant une fonction fluorescente peuvent également être utilisés dans le cadre de l'invention. Un monomère présentant une fonction fluorescente peut être détecté par n'importe quelle méthode appropriée, par exemple par fluorométrie avec un fluorimètre à longueur d'onde fixe. Généralement, la détection du monomère présentant une fonction fluorescente se fait aux maxima d'excitation et d'émission, qui peuvent être déterminés à l'aide d'un fluorimètre à balayage.

Les monomères présentant une fonction fluorescente sont choisis, par exemple, parmi les monomères de type styrène sulfonate de sodium ou styrène sulfonique.

Le (co)polymère hydrosoluble est préférentiellement un (co)polymère anionique à base d'acrylamide et d'acide 2-acrylamido-2-méthylpropane sulfonique ; 50% à 100% de l'acide 2-acrylamido-2-méthylpropane sulfonique étant sous la forme cristalline hydratée, optionnellement partiellement post-hydrolysé, plus préférentiellement un terpolymère d'acrylamide, d'acide acrylique et d'acide 2-acrylamido-2-méthylpropane sulfonique, ou d'au moins un de leurs sels ; 50% à 100% de l'acide 2-acrylamido-2-méthylpropane sulfonique étant sous la forme cristalline hydratée. Dans les deux cas, le (co)polymère peut être partiellement ou totalement post hydrolysé, les monomères anioniques pouvant être sous forme acide ou salifiée.

Le (co)polymère hydrosoluble est préférentiellement obtenu à partir d'entre 10% et 100% molaire de monomère(s) anionique(s), plus préférentiellement entre 20% et 100% molaire, ces pourcentages incluant le monomère correspondant à la forme cristalline hydratée de l'acide 2-acrylamido-2-méthylpropane sulfonique ou un de ses sels.

Le (co)polymère hydrosoluble est préférentiellement obtenu à partir d'entre 10% et 100% molaire de la forme cristalline hydratée de l'acide 2-acrylamido-2-méthylpropane sulfonique.

De manière préférée, le (co)polymère hydrosoluble contient uniquement des unités monomériques anioniques et non-ioniques. En d'autres termes, il est préférentiellement obtenu à partir d'au moins un monomère anionique et d'au moins un monomère non-ionique.

Selon l'invention, le (co)polymère hydrosoluble peut avoir une structure linéaire, branché, star (en forme d'étoile), comb (en forme de peigne) ou bloc. Ces structures peuvent être obtenues par sélection au choix de l'amorceur, de l'agent de transfert, de la technique de polymérisation telle que la polymérisation radicalaire contrôlée dite RAFT (transfert de chaîne réversible par addition-fragmentation, de l'anglais « reversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais « Atom Transfert Radical Polymerization »), de l'incorporation de monomères structuraux, de la concentration...

De manière générale, le (co)polymère ne nécessite pas de développement de procédé de polymérisation particulier. En effet, il peut être obtenu selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; ou de polymérisation micellaire.

Selon un mode particulier de l'invention, le (co)polymère peut être post hydrolysé. La post hydrolyse est la réaction du (co)polymère après polymérisation. Cette étape consiste en la réaction de groupes fonctionnels hydrolysables de monomères avantageusement non ioniques, plus avantageusement les fonctions amide ou ester, avec un agent d'hydrolyse. Cet agent d'hydrolyse peut être une enzyme, une résine échangeuse d'ion, ou un métal alcalin. Préférentiellement, l'agent d'hydrolyse est une base. Durant cette étape de post-hydrolyse du copolymère, le nombre de fonctions acide carboxylique augmente. En effet, la réaction entre la base et les fonctions amides ou esters présentes dans le copolymère produit des groupes carboxylates.

Selon l'invention, le (co)polymère peut se présenter sous forme liquide, gel ou solide lorsque sa préparation inclut une étape de séchage tel que le « spray drying » (séchage par pulvérisation), le séchage sur tambour le séchage par rayonnement tel que le séchage micro-ondes, ou encore le séchage en lit fluidisé.

Selon l'invention, le (co)polymère hydrosoluble est linéaire ou structuré. Par (co)polymère structuré, on désigne un (co)polymère non linéaire qui possède des chaînes latérales de manière à obtenir, lorsque ce (co)polymère est mis en solution dans l'eau, un fort état d'enchevêtrement conduisant à des viscosités à bas gradient très importantes. Le (co)polymère hydrosoluble, selon l'invention n'est généralement pas réticulé.

Le (co)polymère hydrosoluble peut en outre être structuré :

- par au moins un agent de structure, pouvant être choisi dans le groupe comprenant des monomères à insaturation polyéthylénique (ayant au minimum deux fonctions insaturées), comme par exemple les fonctions vinyliques, allyliques, acryliques et époxy et l'on peut citer par exemple le méthylène bis acrylamide (MBA), la triallyamine, le chlorure de tétraallylammonium, ou le 1,2 dihydroxyethylène bis-(N-acrylamide), et/ou
- par des macroamorceurs tels que les polyperoxydes, polyazoïques et les polyagents de transfert tels que les (co)polymères polymercaptants, et les polyols, et/ou
- par des polysaccharides fonctionnalisés.

La quantité d'agent de ramification/réticulation dans le mélange de monomères est avantageusement inférieure à 4 % en poids par rapport à la teneur en monomères, plus avantageusement inférieure à 1 %, et encore plus avantageusement inférieure à 0,5 %. Selon un mode de réalisation particulier, elle peut être au moins égale à 0,00001 % en poids par rapport à la teneur en monomères.

Selon un mode de réalisation particulier, le (co)polymère hydrosoluble peut comprendre au moins un groupe à LCST.

Selon les connaissances générales de l'homme du métier, un groupe à LCST correspond à un groupe dont la solubilité dans l'eau pour une concentration déterminée, est modifiée au-delà d'une certaine température et en fonction de la salinité. Il s'agit d'un groupe présentant une température de transition par chauffage définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition minimale est appelée « LCST » (température critique inférieure de solubilité, de l'anglais « Lower Critical Solution Température »). Pour chaque concentration de groupe à LCST, une température de transition par chauffage est observée. Elle est supérieure à la LCST qui est le point minimum de la courbe. En dessous de cette température, le (co)polymère est soluble dans l'eau, au-dessus de cette température, le (co)polymère perd sa solubilité dans l'eau.

Selon un mode de réalisation particulier, le (co)polymère hydrosoluble peut comprendre au moins un groupe à UCST.

Selon les connaissances générales de l'homme du métier, un groupe à UCST correspond à un groupe dont la solubilité dans l'eau pour une concentration déterminée, est modifiée en dessous d'une certaine température et en fonction de la salinité. Il s'agit d'un groupe présentant une température de transition par refroidissement définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition maximale est appelée « UCST » (température critique supérieure de solubilité, de l'anglais « Upper Critical Solution Température »). Pour chaque concentration de groupe à UCST, une température de transition par refroidissement est observée. Elle est inférieure à la UCST qui est le point maximum de la courbe. Au-dessus de cette température, le (co)polymère est soluble dans l'eau, en-dessous de cette température, le (co)polymère perd sa solubilité dans l'eau.

Selon l'invention, le (co)polymère a un poids moléculaire avantageusement élevé. Par « poids moléculaire élevé », on désigne des poids moléculaires d'au moins 1 million de g/mol, préférentiellement entre 2 et 40 millions g/mol, plus préférentiellement entre 5 et 30 millions g/mol. Le poids moléculaire s'entend en poids moléculaire moyen en poids.

Selon l'invention, le (co)polymère a un quotient de filtration (ou filter ratio noté « FR ») inférieur à 1,5, préférentiellement inférieur à 1,3 plus préférentiellement inférieur à 1,1.

Le terme quotient de filtration est utilisé dans le présent document pour désigner un test utilisé pour déterminer la performance de la solution de polymère dans des conditions approchant la perméabilité du gisement consistant à mesurer le temps pris par des volumes/concentrations donnés de solution pour traverser un filtre. Le FR compare généralement la filtrabilité de la solution polymère pour deux volumes consécutifs équivalents, ce qui indique la tendance de la solution à boucher le filtre. Les FR plus faibles indiquent un meilleur rendement.

Le test utilisé pour déterminer le FR consiste à mesurer les temps que mettent des volumes donnés de solution à 1000 ppm actif de polymère pour s'écouler au travers d'un filtre. La solution est contenue dans une cellule pressurisée à deux bars de pression et le filtre est de 47 mm de diamètre et d'une taille de pores définies. Généralement le FR est mesuré avec des filtres ayant une taille de pores de 1,2 $\mu$m, 3 $\mu$m, 5 $\mu$m ou 10 $\mu$m.

Les temps nécessaires pour obtenir 100 ml ($t_{100ml}$) ; 200 ml ($t_{200ml}$) et 300 ml ($t_{300ml}$) de filtrat sont donc mesurés et est défini alors un FR s'exprimant par :

$$FR = \frac{t_{300\,ml} - t_{200\,ml}}{t_{200\,ml} - t_{100\,ml}}$$

Les temps sont mesurés à 0,1 seconde près.

Le FR représente ainsi la capacité de la solution de polymère à colmater le filtre pour deux volumes consécutifs équivalents.

Les (co)polymères utilisés selon l'invention, présentent une résistance à la dégradation chimique et à la dégradation thermique améliorée par rapport à des (co)polymères de poids moléculaire équivalents fabriqués à partir d'acide 2-acrylamido-2-méthylpropane sulfonique n'étant pas sous forme cristalline hydratée.

Le test utilisé pour déterminer la résistance à la dégradation chimique consiste à préparer une solution de polymère à une concentration donnée dans une saumure donnée en conditions aérobiques et de la mettre en contact avec un contaminant chimique tel que le fer ou le sulfure d'hydrogène. La viscosité de la solution de polymère est mesurée avant et après 24 h d'exposition au contaminant. Les mesures de viscosité sont réalisées dans les mêmes conditions de température et de gradient de cisaillement.

Le test utilisé pour déterminer la résistance à la dégradation mécanique consiste à préparer une solution de polymère à une concentration donnée dans une saumure de composition donnée en conditions anaérobiques (utilisation d'une boîte à gant inertée à l'azote) et à la laisser vieillir dans une cellule en acier inoxydable placée à une température donnée pendant un temps prédéfini. A échéance, la cellule en acier inoxydable est refroidie à température ambiante puis la viscosité de la solution de polymère qu'elle contient est mesurée et comparée à sa valeur initiale. Toute manipulation de la cellule en acier inoxydable est réalisée dans l'enceinte de la boite à gants pour éviter toute exposition à l'oxygène. Les cellules en acier inoxydable sont étanches pour prévenir toute entrée d'oxygène dans la solution lors du vieillissement en température. Les mesures de viscosité avant et après vieillissement sont réalisées en boite à gant dans les mêmes conditions de température et de gradient de vitesse.

La résistance à la dégradation chimique et à la dégradation thermique est quantifiée par la valeur de perte de viscosité exprimée en pourcent et déterminée à échéance par :

$$Perte\ de\ viscosit\acute{e}\ (\%) = \frac{Viscosit\acute{e}_{initiale} - Viscosit\acute{e}_{\grave{a}\ \acute{e}ch\acute{e}ance}}{Viscosit\acute{e}_{initiale}} \times 100$$

Plus précisément, la présente invention concerne également un procédé de récupération assistée du pétrole et/ou du gaz utilisant au moins un (co)polymère hydrosoluble fabriqué à partir de la forme cristalline hydratée de l'acide 2-acrylamido-2-méthylpropane sulfonique ou d'au moins un de ses sels ci-dessus mentionnés (sels de métaux alcalins, de métaux alcalino-terreux, ou d'ammonium).

Comme déjà indiqué, l'invention concerne un procédé de récupération assistée du pétrole caractérisé en ce qu'il comprend les étapes suivantes :

a) Préparation d'un fluide d'injection comprenant au moins un (co)polymère hydrosoluble fabriqué au moins à partir de la forme cristalline hydratée de l'acide 2-acrylamido-2-méthylpropane sulfonique ou d'au moins un de ses sels, avec de l'eau ou de la saumure,
b) Injection du fluide d'injection dans une formation souterraine,
c) Balayage de la formation souterraine à l'aide du fluide injecté,
d) Récupération d'un mélange aqueux et hydrocarboné.

Lorsque le (co)polymère hydrosoluble est sous forme de particules, il peut être dissous dans un milieu aqueux dans un dispositif de dispersion. Un exemple de dispositif de dispersion est l'unité de tranchage de polymères (PSU) décrite dans le document US 8,186,871, qui permet la préparation d'une solution aqueuse concentrée en polymère.

L'eau ou la saumure utilisée pour la préparation du fluide d'injection peut être une eau de production. On entend par « eau de production » toutes les eaux salées ou non salées, saumures, eau de mer, eau d'aquifère qui sont issues d'un réservoir d'hydrocarbures. Cette eau de production peut préalablement être traitée avant la préparation du fluide d'injection comme décrit dans la demande de brevet WO2018/020175.

Les (co)polymères hydrosolubles peuvent être associés avec des composés stabilisants. Les composés stabilisants (agents stabilisants) peuvent être des composés qui protègent convenablement les (co)polymères, par exemple contre la dégradation thermique, chimique et/ou mécanique. Des exemples d'agents stabilisants appropriés sont fournis dans la demande de brevet WO2010/133258,

L'injection du fluide d'injection comprenant le (co)polymère hydrosoluble, selon la technique employée, se fait seule ou en conjonction avec un ou plusieurs composés chimiques utiles à la récupération améliorée du pétrole. Parmi ces composés chimiques, on citera l'utilisation de base faible, forte ou super-forte, minérale ou organique pouvant saponifier les bruts et fabriquer in-situ des espèces tensioactives solubilisantes du pétrole. A titre d'exemple, on trouve parmi ceux-ci le carbonate de sodium, la soude caustique, les composés borates et metaborates, les amines, les espèces polymériques basiques. Une autre famille de composés largement injectée avec les polymères est celle des composés tensioactifs, souvent anioniques, zwiterrioniques, cationiques et parfois aussi non ioniques. Ces composés sont rarement injectés purs mais avec un cotensioactif et un co-solvant pour améliorer leur compatibilité et leur efficacité dans le réservoir.

Selon l'invention, le fluide d'injection comprend avantageusement entre 10 ppm et 15 000 ppm de (co)polymère hydrosoluble, plus avantageusement entre 50 et 10 000 ppm, et encore plus avantageusement entre 100 et 5 000 ppm.

De manière totalement surprenante, la Demanderesse a découvert que les (co)polymères hydrosolubles fabriqués à partir de la forme cristalline hydratée de l'acide 2-acrylamido-2-méthylpropane sulfonique ou d'au moins un de ses sels, ont une meilleure filtrabilité ainsi qu'une meilleure résistance à la dégradation chimique et à la dégradation thermique par rapport à des (co)polymères de poids moléculaire équivalents réalisés à partir d'acide 2-acrylamido-2-méthylpropane sulfonique n'étant pas sous forme cristalline hydratée. Les (co)polymères de l'invention présentent une filtrabilité améliorée par rapport à des (co)polymères de poids moléculaire équivalents réalisés avec l'acide 2-acrylamido-2-méthylpropane sulfonique n'étant pas sous forme hydratée et cristalline. Par ailleurs, il est connu que la filtrabilité se détériore lorsque le poids moléculaire du (co)polymère augmente. L'intérêt de l'invention est que des (co)polymères de très haut poids moléculaire peuvent être obtenues tout en ayant une bonne filtrabilité. De plus la concentration en polymère nécessaire pour atteindre une viscosité de fluide d'injection cible se trouve réduite, ce qui améliore les conditions économiques de la récupération de l'hydrocarbure et/ou du gaz contenu(s) dans la formation souterraine.

Les (co)polymères hydrosolubles selon l'invention ont pour fonctionnalité de viscosifier les eaux injectées dans les réservoirs contenant du pétrole ou du gaz pour assurer un contrôle de mobilité sans qu'un recours à la réticulation, c'est à dire un pontage chimique inter chaines, ne soit nécessaire.

Selon un mode particulier de l'invention, le procédé de récupération assistée du pétrole est caractérisé en ce qu'il comprend les étapes suivantes :

a) Préparation d'un fluide d'injection comprenant des (co)polymères ayant un poids moléculaire supérieure à 5 millions caractérisé en ce que

- le fluide d'injection a une concentration en sel(s) supérieure à 100g/l dont au plus 50g/l de sel(s)s divalent(s),
- les (co)polymères sont obtenus au moins à partir d'au moins 80% mol d'ATBS, 50% à 100% étant sous forme cristalline hydratée et/ou au moins un de ses sels,
- la concentration en polymère du fluide d'injection est inférieure à 3000ppm en poids,
- le fluide d'injection avant l'étape de cisaillement b) a une viscosité $V_1$,

b) Cisaillement du fluide d'injection pour obtenir une baisse de la viscosité de plus de 25% par rapport à $V_1$ et

caractérisé en ce que

$$\frac{V2-Veau}{Veau} \geq 3$$

où,

V$_2$ est la viscosité du fluide d'injection après cisaillement à la température de la formation,
V$_{eau}$ est la viscosité de l'eau utilisée pour la préparation du fluide d'injection à la température de la formation,

c) Injection du fluide d'injection dans une formation souterraine caractérisée en ce que la formation souterraine est une formation carbonatée, ayant une perméabilité inférieure à 300 millidarcy et une température supérieure à 100°C,
d) Balayage de la formation souterraine à l'aide du fluide injecté,
e) Récupération d'un mélange aqueux et hydrocarboné,

V1 correspond à la viscosité du fluide d'injection avant l'étape de cisaillement à la température de la formation.

Comme déjà indiqué, V2 correspond à la viscosité du fluide d'injection après l'étape de cisaillement à la température de la formation. Veau correspond à la viscosité de l'eau utilisée pour la préparation du fluide d'injection à la température de la formation.

L'étape de cisaillement peut être effectuée, par exemple, à l'aide d'une vanne, d'un orifice ou d'une pompe.

Préférentiellement, la concentration en sel(s) divalent(s) dans le fluide d'injection est comprise entre 3 et 50g/l.

Les formations carbonatées sont des formations de roches sédimentaires dont la composition en carbonates est d'au moins 50 %.

L'invention et les avantages qui en découlent ressortiront mieux des figures et exemples suivants donnés afin d'illustrer l'invention et non de manière limitative.

DESCRIPTION DES FIGURES

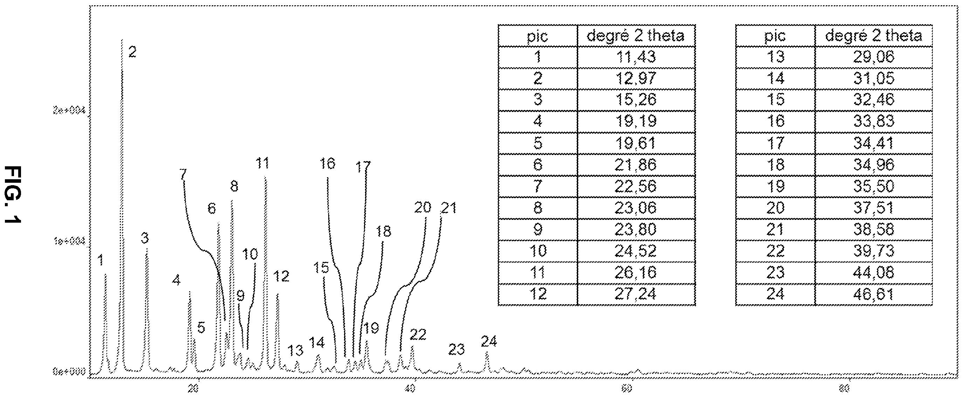

La figure 1 illustre le diagramme par diffraction des rayons X des cristaux obtenus selon l'exemple 1.
La figure 2 illustre le diagramme par diffraction des rayons X des cristaux obtenus selon l'exemple 2.
La figure 3 illustre le spectre infra-rouge à transformée de Fourier des cristaux obtenus en exemple 1.
La figure 4 illustre le spectre infra-rouge à transformée de Fourier des cristaux obtenus selon l'exemple 2.
La figure 5 illustre le quotient de filtration en fonction de la forme de l'ATBS et du poids moléculaire de copolymères.
La figure 6 illustre la perte de viscosité en fonction de la forme de l'ATBS et de la teneur en fer de copolymères.
La figure 7 illustre la perte de viscosité en fonction de la forme de l'ATBS en vieillissement à 90°C de copolymères.
La figure 8 illustre le quotient de filtration en fonction de la forme de l'ATBS et du poids moléculaire d'homopolymères
La figure 9 illustre la perte de viscosité en fonction de la forme de l'ATBS et de la teneur en fer d'homopolymères.
La figure 10 illustre le quotient de filtration en fonction de la forme de l'ATBS et du poids moléculaire de copolymères post-hydrolysé.

EXEMPLES DE REALISATION DE L'INVENTION

Exemple 1 : Synthèse de l'acide 2-acrylamido-2-méthylpropane sulfonique

Dans un réacteur agité de 2000 ml ayant une double enveloppe, sont ajoutés 1522 grammes d'acrylonitrile contenant 0,4% d'eau en poids et 180 grammes d'acide sulfurique fumant titrant 104% H$_2$SO$_4$ (18% Oléum). Le mélange est agité pendant 1 heure et refroidi par la double enveloppe du réacteur qui maintient la température du mélange sulfonant à -20°C.

97 grammes d'isobutylène sont additionnés au mélange sulfonant précédent, à un débit de 1,6 grammes/minute.

La température du mélange est contrôlée à 45°C lors de l'introduction de l'isobutylène. Les particules de l'acide 2-acrylamido-2-méthylpropane sulfonique précipitent dans le mélange et le taux de solide est d'environ 20% en poids. Le mélange réactionnel est filtré sur un filtre de type Büchner et séché sous vide à 50°C. Le solide obtenu est l'acide 2-acrylamido-2-méthylpropane sulfonique et se présente sous forme de poudre blanche très fine.

Exemple 2 : Synthèse de la forme cristalline hydratée de l'acide 2-acrylamido-2-méthylpropane sulfonique

Dans un réacteur agité de 2000 ml ayant une double enveloppe, sont ajoutés 500 grammes d'acide 2-acrylamido-2-méthylpropane sulfonique obtenu à l'exemple 1 et 460 grammes d'acide sulfurique à une concentration de 10% en $H_2SO_4$.

250 mg de 4-hydroxy-2,2,6,6-tétraméthylpipéridin-1-oxyl sont ajoutés au mélange précédant.

Le mélange est agité pendant 10 minutes, à 20°C, pour former une suspension A.

La suspension A est chauffée à une température de 60°C et est maintenue à cette température pendant 20 minutes afin de former une solution B.

La solution B est refroidie à une température de 10°C. Le temps de refroidissement entre 60°C et 10°C est de 6 heures. Une suspension C de cristaux d'acide 2-acrylamido-2-méthylpropane sulfonique est obtenue. La suspension C est filtrée sur une essoreuse verticale de marque Robatel. Un solide de composition 1 est obtenu, il contient 80% en poids de cristaux d'acide 2-acrylamido-2-méthylpropane sulfonique.

Exemple 3 : Analyse par diffraction aux rayons X

Les solides obtenus aux exemples 1 et 2 sont préalablement broyés pour former des poudres et sont analysés par diffraction aux rayons X sur une gamme angulaire de 10 à 90°. L'équipement utilisé est un diffractomètre miniflex II de marque Rigaku et est équipé d'une source au cuivre.

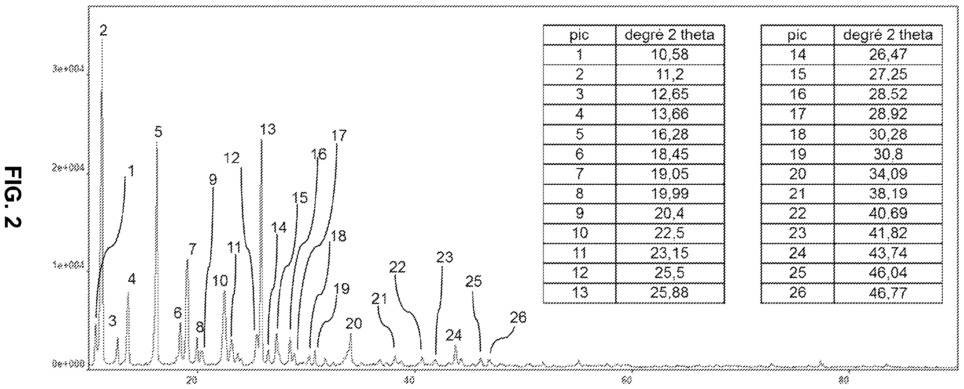

Nous pouvons observer que le solide obtenu à l'issu de l'exemple 2 (figure 2) a un diagramme de diffraction aux rayons X ayant les pics caractéristiques suivants :
10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta (+/-0.1°).

Exemple 4 : Mesure infra-rouge à transformée de Fourier

L'équipement de mesure infra-rouge par transformée de Fourier est le Spectrum 100 de la marque Perkin Elmer, dont la précision est de 8cm$^{-1}$.

Les solides obtenus aux exemples 1 et 2 sont tamisés à 100$\mu$m. Les particules restant sur le tamis sont séchées et mises à l'étuve à 60°C pendant au moins 4 heures.

10 mg de solide sont précisément pesés et sont mélangés avec 500 mg de bromure de potassium (KBr). Le mélange est ensuite compacté dans une presse hydraulique sous une pression d'au moins 10 bars.

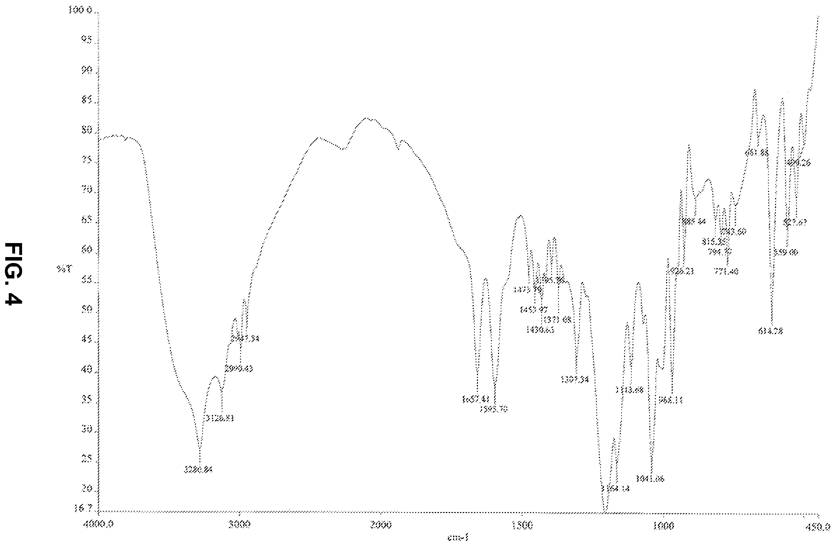

Nous pouvons observer que les bandes suivantes (figure 4) sont caractéristiques de la forme cristalline hydratée de l'acide 2-acrylamido-2-méthylpropane sulfonique : 3280cm$^{-1}$, 3126cm$^{-1}$, 1657cm$^{-1}$, 1595cm$^{-1}$, 1453cm$^{-1}$, 1395cm$^{-1}$, 1307cm$^{-1}$, 1205cm$^{-1}$, 1164cm$^{-1}$, 1113cm$^{-1}$, 1041cm$^{-1}$, 968cm$^{-1}$, 885cm$^{-1}$, 815cm$^{-1}$, 794cm$^{-1}$.

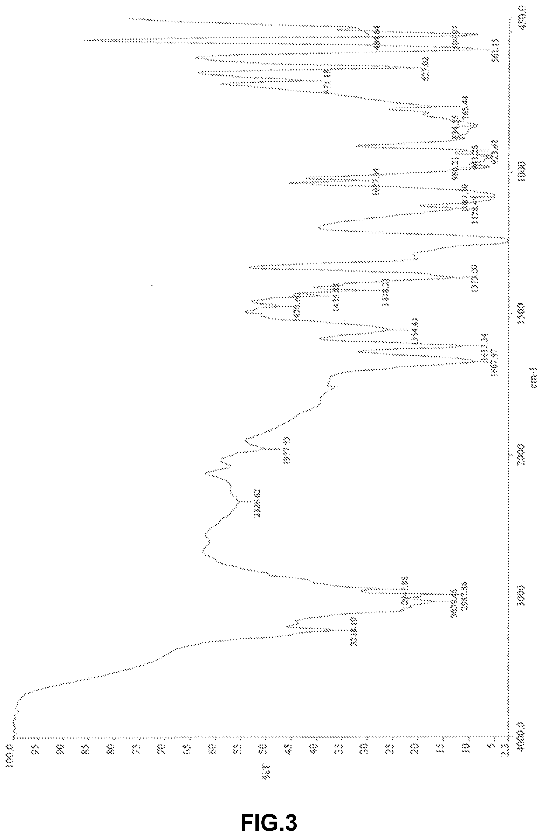

Le spectre infra-rouge du solide selon l'exemple 1 (figure 3) ne présente pas les mêmes pics.

Exemple 5 : Préparation du copolymère acrylamide/acide 2-acrylamido-2-méthylpropane sulfonique de forme cristalline hydratée (75/25 mole%)

Dans un bécher de 2000 ml sont ajoutés 549,5 g d'eau déionisée, 520.5 g d'acrylamide en solution à 50%, 97,6 g de lessive de soude à 50%, 16,2 g d'urée et 316,2 g de cristaux d'acide 2-acrylamido-2-méthylpropane sulfonique obtenus dans l'exemple 2.

La solution ainsi obtenue est refroidie entre 5 et 10°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :

• 0,45 g de 2,2'-azobisisobutyronitrile,
• 1,5 ml d'une solution à 2,5 g/l de dihydrochlorure de 2,2'-azobis[2-(2-imidazolin-2-yl)propane],
• 1,5 ml d'une solution à 1 g/l d'hypophosphite de sodium,
• 1,5 ml d'une solution à 1 g/l de ter-butyl hydroperoxyde,
• 1,5 ml d'une solution à 1 g/l de sulfate d'ammonium et de fer(II) hexahydraté (sel de Mohr).

Après quelques minutes, l'arrivée d'azote est fermée et le réacteur est clos. La réaction de polymérisation se déroule pendant 2 à 5 heures jusqu'à atteindre un pic de température. Le gel caoutchouteux obtenu est haché et séché pour obtenir une poudre grossière elle-même broyée et tamisée pour obtenir le polymère sous forme de poudre.

Exemple 6 : Préparation du copolymère acrylamide/acide 2-acrylamido-2-méthylpropane sulfonique de forme non cristalline hydratée (75/25 mole%)

Les polymères sont préparés comme dans l'exemple 5 en remplaçant l'acide 2-acrylamido-2-méthylpropane sulfonique de forme cristalline hydratée (exemple 2) par de l'acide 2-acrylamido-2-méthylpropane sulfonique n'étant pas de forme cristalline hydratée synthétisé en exemple 1.

Exemple 7 : Mesure du quotient de filtration des solutions de polymères

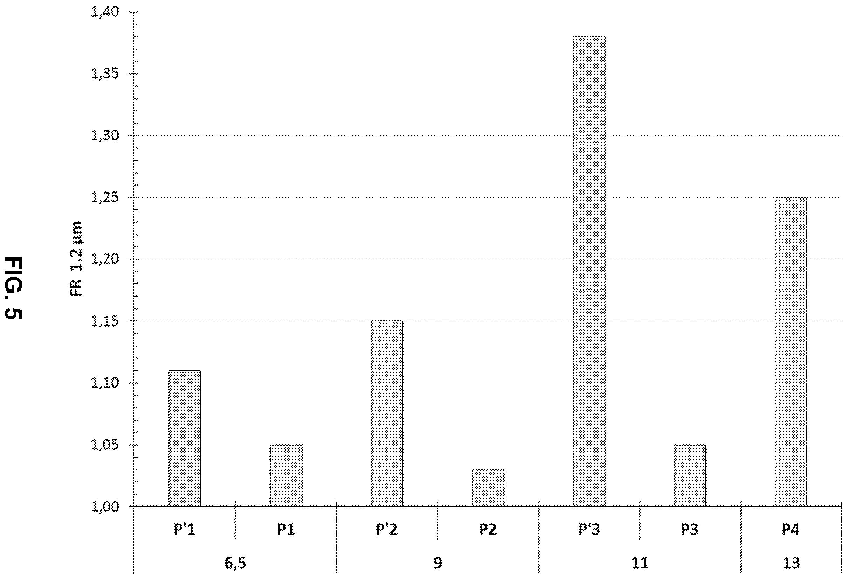

Des tests de filtration ont été réalisés sur 3 polymères préparés à partir de la forme non cristalline de l'acide 2-acrylamido-2-méthylpropane sulfonique P'1, P'2 et P'3 de poids moléculaire croissants respectifs 6,5, 9 et 11,5 millions Da, préparés comme décrit dans l'exemple 6, et sur 4 polymères préparés à partir de la forme cristalline de l'acide 2-acrylamido-2-méthylpropane sulfonique P1, P2, P3 et P4 de poids moléculaire croissants respectifs 6,5, 9, 11 et 13 millions DA, préparés comme décrit dans l'exemple 5. Le grade de poids moléculaire 13 millions Da n'est pas accessible lors de l'utilisation de la forme non cristalline de l'acide 2-acrylamido-2-méthylpropane sulfonique.
Les solutions de polymères ont été préparées à une concentration active de 1 000 ppm dans une saumure contenant de l'eau, 30 000 ppm de NaCl et 3 000 ppm de $CaCl_2,2H_2O$. Le quotient de filtration (FR) a été mesuré sur des filtres ayant une taille de pore de 1,2 $\mu$m représentatifs de gisements de faible perméabilité. Les résultats sont présentés sur la figure 5.

Tableau 1 : Polymères testés pour le quotient de filtrabilité

| | Forme d'ATBS utilisée | Poids moléculaire (en millions Da) | Quotient de filtration |
|---|---|---|---|
| P1 | Cristalline | 6,5 | 1.05 |
| P2 | Cristalline | 9 | 1.03 |
| P3 | Cristalline | 11 | 1.05 |
| P4 | Cristalline | 13 | 1.25 |
| P'1 | Non cristalline | 6,5 | 1.11 |
| P'2 | Non cristalline | 9 | 1.15 |
| P'3 | Non cristalline | 11,5 | 1.38 |

Nous pouvons observer qu'à poids moléculaire équivalents les polymères préparés à partir de la forme cristalline de l'acide 2-acrylamido-2-méthylpropane sulfonique (P1-P3) présentent toujours un FR inférieur à celui des polymères préparés à partir de la forme non cristalline de l'acide 2-acrylamido-2-méthylpropane sulfonique (P'1-P'3). Cet écart devient de plus en plus important quand le poids moléculaire du polymère augmente. Le polymère fabriqué à partir de la forme cristalline de l'acide 2-acrylamido-2-méthylpropane sulfonique de poids moléculaire 13 millions Da (P4) présentent même un FR plus faible que le polymère préparé à partir de la forme non cristalline de l'acide 2-acrylamido-2-méthylpropane sulfonique de poids moléculaire inférieur (11,5 millions Da, P'3).

Exemple 8 : Mesure de la résistance à la dégradation chimique de solutions de polymères de poids moléculaire équivalents

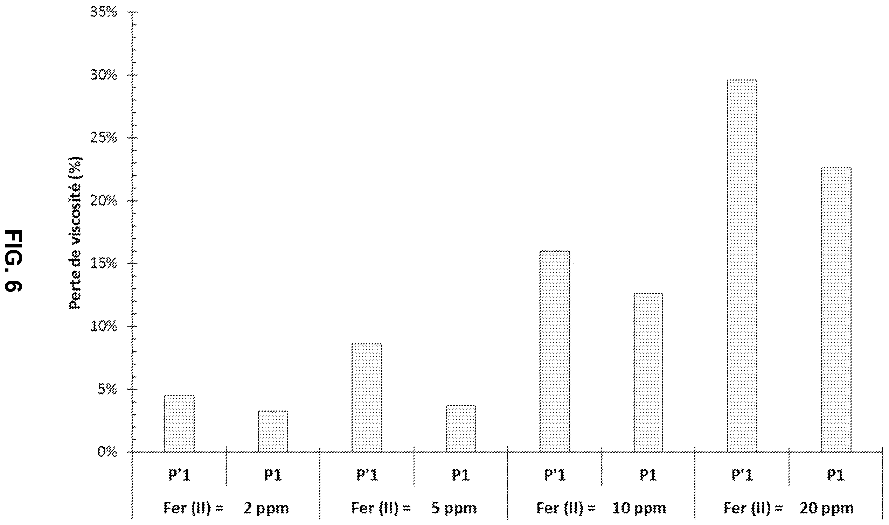

Des tests de résistance à la dégradation chimique des polymères P3 et P'3 ont été réalisés en conditions aérobiques en présence de différentes concentrations en fer(II) (2, 5, 10 et 20 ppm) dans une saumure composée d'eau, de 37 000 ppm de NaCl, 5 000 ppm de $Na_2SO_4$ et 200 ppm de $NaHCO_3$. Ces tests ont été réalisés sur un polymère préparé à partir de la forme non cristalline de l'acide 2-acrylamido-2-méthylpropane sulfonique (P'3) et sur un polymère fabriqué à partir de la forme cristalline de l'acide 2-acrylamido-2-méthylpropane sulfonique (P3). Les deux polymères ont la même composition chimique. Les résultats obtenus après 24 h de mise en contact de la solution de polymère avec le contaminant sont présentés sur la figure 6.
Nous pouvons observer que pour chaque concentration en fer(II) le polymère P3 perd moins de viscosité que le polymère P'3 équivalent.

Exemple 9 : Mesure de la résistance à la dégradation thermique de solutions de polymères de poids moléculaire équivalents

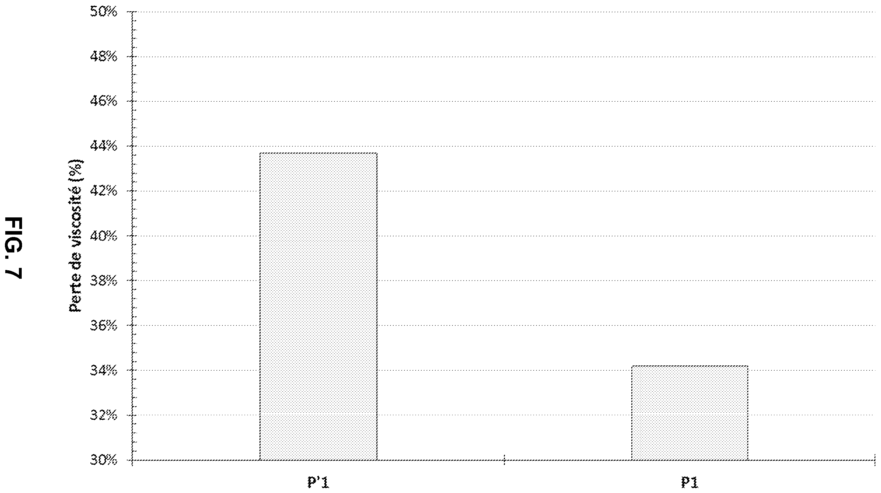

Des tests de résistance à la dégradation thermique des polymères P3 et P'3 ont été réalisés en conditions anaérobiques à une concentration active de 2 000 ppm dans une saumure composée de 30 000 ppm de NaCl et 3 000 ppm de CaCl$_2$,2H$_2$O. Ces tests ont été réalisés sur un polymère préparé à partir de la forme non cristalline de l'acide 2-acrylamido-2-méthylpropane sulfonique (P'3) et sur un polymère fabriqué à partir de la forme cristalline de l'acide 2-acrylamido-2-méthylpropane sulfonique (P3). Les deux polymères ont la même composition chimique. Les solutions de polymères ont été vieillies pendant 6 mois à 90°C. Les résultats obtenus sont présentés sur la figure 7 en termes de perte de viscosité. Nous pouvons observer que le P3 perd moins de viscosité que le P'3 équivalent.

Exemple 10 : Préparation d'homopolymères à partir de la forme cristalline hydratée de l'acide 2-acrylamido-2-méthylpropane sulfonique

Dans un bécher de 2000 ml sont ajoutés 390,5 g d'eau déionisée, 262 g de lessive de soude à 50% et 847,5 g de cristaux d'acide 2-acrylamido-2-méthylpropane sulfonique obtenus dans l'exemple 2.
La solution ainsi obtenue est refroidie entre 5 et 10°C et transférée dans un réacteur de polymérisation adiabatique, un bullage d'azote est réalisé pendant 30 minutes afin d'éliminer toute trace d'oxygène dissout.
Sont ensuite ajoutés dans le réacteur :

- 0,45 g de 2,2'-azobisisobutyronitrile,
- 1,5 ml d'une solution à 2,5 g/l de dihydrochlorure de 2,2'-azobis[2-(2-imidazolin-2-yl)propane],
- 1,5 ml d'une solution à 1 g/l d'hypophosphite de sodium,
- 1,5 ml d'une solution à 1 g/l de ter-butyl hydroperoxyde,
- 1,5 ml d'une solution à 1 g/l de sulfate d'ammonium et de fer(II) hexahydraté (sel de Mohr).

Après quelques minutes, l'arrivée d'azote est fermée et le réacteur est clos. La réaction de polymérisation se déroule pendant 2 à 5 heures jusqu'à atteindre un pic de température. Le gel caoutchouteux obtenu est haché et séché pour obtenir une poudre grossière elle-même broyée et tamisée pour obtenir le polymère sous forme de poudre.

Exemple 11: Préparation d'homopolymères à partir de l'acide 2-acrylamido-2-méthylpropane sulfonique de forme non cristalline hydratée.

Les polymères sont préparés comme dans l'exemple 10 en remplaçant l'acide 2-acrylamido-2-méthylpropane sulfonique de forme cristalline hydratée (exemple 2) par de l'acide 2-acrylamido-2-méthylpropane sulfonique n'étant pas de forme cristalline hydratée synthétisé en exemple 1.

Exemple 12 : Mesure du quotient de filtration des solutions de polymères

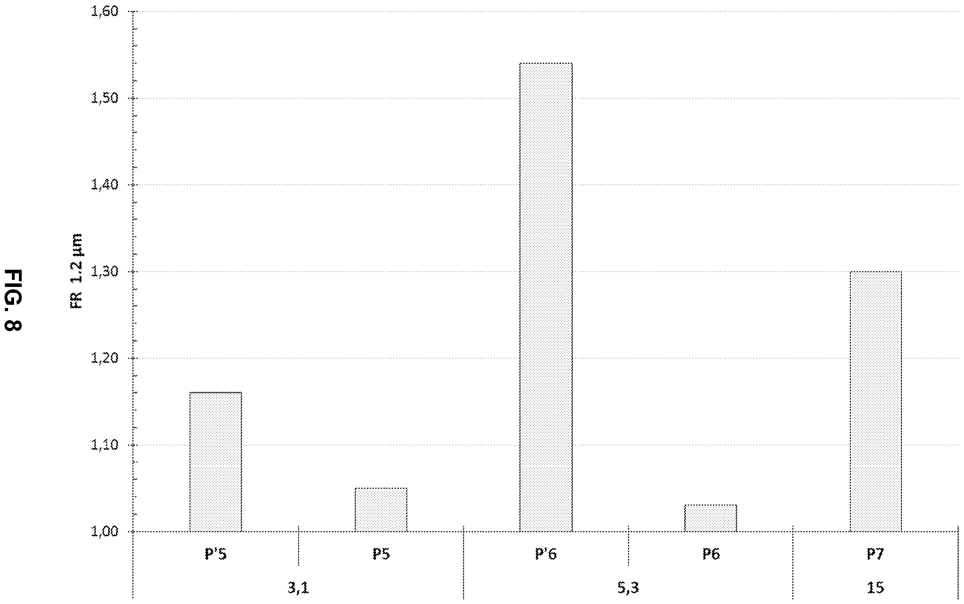

Des tests de filtration ont été réalisés sur 2 polymères préparés à partir de la forme non cristalline de l'acide 2-acrylamido-2-méthylpropane sulfonique P'5, P'6 de poids moléculaire croissants respectifs 3,1 et 5,3 millions Da, préparés comme décrit dans l'exemple 11, et sur 3 polymères fabriqués à partir de la forme cristalline de l'acide 2-acrylamido-2-méthylpropane sulfonique P5, P6 et P7 de poids moléculaire croissants respectifs 3,1, 5,3 et 15 millions Da, préparés comme décrit dans l'exemple 10. Le grade de poids moléculaire 15 millions Da n'est pas accessible lors de l'utilisation de la forme non cristalline de l'acide 2-acrylamido-2-méthylpropane sulfonique.
Les solutions de polymères ont été préparées à une concentration active de 1 000 ppm dans une saumure contenant de l'eau, 30 000 ppm de NaCl et 3 000 ppm de CaCl$_2$,2H$_2$O. Le quotient de filtration (FR) a été mesuré sur des filtres ayant une taille de pore de 1,2 $\mu$m représentatifs de gisements de faible perméabilité. Les résultats sont présentés sur la figure 8.

Tableau 2 : Polymères testés pour le quotient de filtrabilité

|  | Forme d'ATBS utilisée | Poids moléculaire (en millions Da) | Quotient de filtration |
|---|---|---|---|
| P5 | Cristalline | 3,1 | 1.05 |
| P6 | Cristalline | 5,3 | 1.03 |
| P7 | Cristalline | 15 | 1.30 |

(suite)

| | Forme d'ATBS utilisée | Poids moléculaire (en millions Da) | Quotient de filtration |
|---|---|---|---|
| P'5 | Non cristalline | 3,1 | 1.16 |
| P'6 | Non cristalline | 5,3 | 1.54 |

Nous pouvons observer qu'à poids moléculaire équivalents les polymères fabriqués à partir de la forme cristalline de l'acide 2-acrylamido-2-méthylpropane sulfonique (P5-P6) présentent toujours un FR inférieur à celui des polymères fabriqués à partir de la forme non cristalline de l'acide 2-acrylamido-2-méthylpropane sulfonique (P'5-P'6). Cet écart devient de plus en plus important quand le poids moléculaire du polymère augmente. Le polymère préparé à partir de la forme cristalline de l'acide 2-acrylamido-2-méthylpropane sulfonique de poids moléculaire 15 millions Da (P7) présentent même un FR plus faible que le polymère préparé à partir de la forme non cristalline de l'acide 2-acrylamido-2-méthylpropane sulfonique de poids moléculaire inférieur (5,3 millions Da, P'6).

Exemple 13 : Mesure de la résistance à la dégradation chimique de solutions des polymères P6 et P'6.

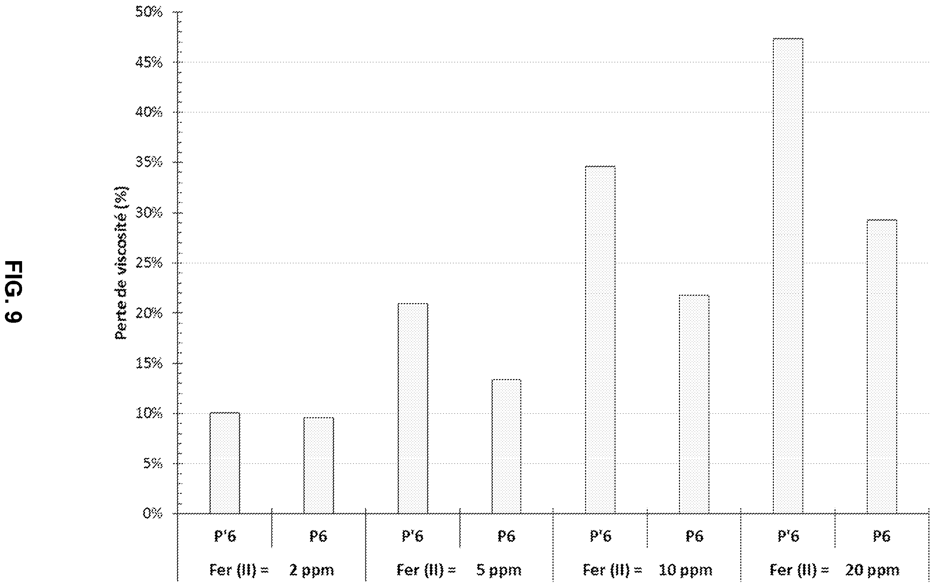

Des tests de résistance à la dégradation chimique des polymères P6 et P'6 de poids moléculaire 5,3 millions Da ont été réalisées en conditions aérobiques en présence de différentes concentrations en fer(II) (2, 5, 10 et 20 ppm) dans une saumure composée d'eau, de 37 000 ppm de NaCl, 5 000 ppm de $Na_2SO_4$ et 200 ppm de $NaHCO_3$. Ces tests ont été réalisés sur un polymère préparé à partir de la forme non cristalline de l'acide 2-acrylamido-2-méthylpropane sulfonique (P'6) et sur un polymère préparé à partir de la forme cristalline de l'acide 2-acrylamido-2-méthylpropane sulfonique (P6). Les deux polymères ont la même composition chimique. Les résultats obtenus après 24 h de mise en contact de la solution de polymère avec le contaminant sont présentés sur la figure 9.

Nous pouvons observer que pour chaque concentration en fer(II) le polymère P6 perd moins de viscosité que le polymère P'6 équivalent.

Exemple 14 : Préparation du copolymère P8 acrylamide/acide 2-acrylamido-2-méthylpropane sulfonique de forme cristalline hydratée (75/25 mole%) post hydrolysé.

Dans un bécher de 2000 ml sont ajoutés 761,9 g d'eau déionisée, 574,2 g d'acrylamide en solution à 50%, 35,9g de lessive de soude à 50%, 11,7g d'urée et 116,3g de cristaux d'acide 2-acrylamido-2-méthylpropane sulfonique obtenus dans l'exemple 2.

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :

- 0,45 g de 2,2'-azobisisobutyronitrile,
- 1,5 ml d'une solution à 5 g/l de dihydrochlorure de 2,2'-azobis[2-(2-imidazolin-2-yl)propane],
- 1,5 ml d'une solution à 1 g/l d'hypophosphite de sodium,
- 2,25 ml d'une solution à 1 g/l de ter-butyl hydroperoxyde,
- 3,0 ml d'une solution à 1 g/l de sulfate d'ammonium et de fer(II) hexahydraté (sel de Mohr).

Après quelques minutes, l'arrivée d'azote est fermée et le réacteur est clos. La réaction de polymérisation se déroule pendant 2 à 5 heures jusqu'à atteindre un pic de température. Le gel caoutchouteux obtenu est haché en particules d'une taille comprise entre 1 et 6 mm.

500,0 g de gel préalablement haché sont ensuite mélangés à 18,0 g de lessive de soude à 50%, le mélange est porté et maintenu à une température de 90°C pour une durée de 90 minutes.

Le gel est ensuite séché et broyé pour obtenir le polymère sous forme de poudre.

Exemple 15 : Préparation du copolymère P'8 acrylamide/acide 2-acrylamido-2-méthylpropane sulfonique de forme non cristalline hydratée (75/25 mole%) post hydrolysé.

Le copolymère est préparé comme dans l'exemple 14 en remplaçant l'acide 2-acrylamido-2-méthylpropane sulfonique de forme cristalline hydratée (exemple 2) par de l'acide 2-acrylamido-2-méthylpropane sulfonique n'étant pas de forme cristalline hydratée obtenue en exemple 1.

Exemple 16 : Mesure du quotient de filtration des solutions de polymères

Des tests de filtration ont été réalisés sur un polymère préparé à partir de la forme non cristalline de l'acide 2-acrylamido-2-méthylpropane sulfonique P'8 de poids moléculaire 22 millions Da, préparés comme décrit dans l'exemple 15, et sur un polymère préparé à partir de la forme cristalline hydratée de l'acide 2-acrylamido-2-méthylpropane sulfonique P8 de poids moléculaire de 26 millions Da, préparés comme décrit dans l'exemple 14.

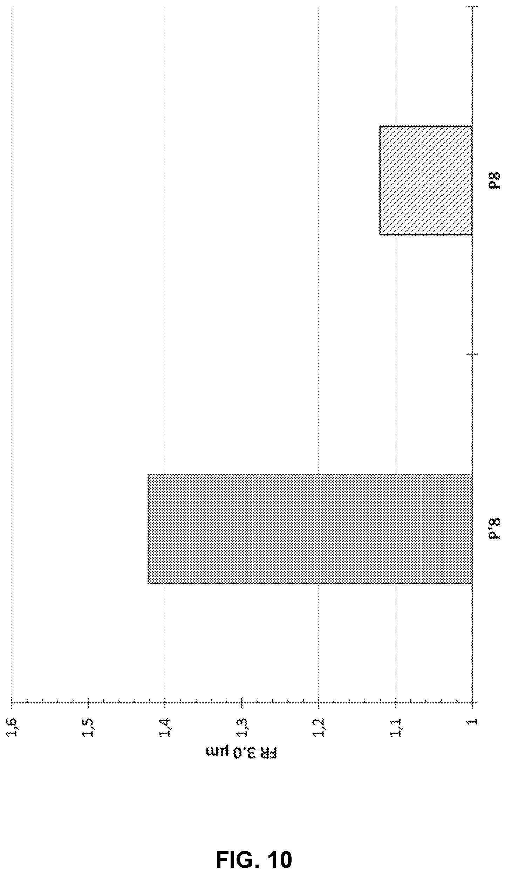

Les solutions de polymères ont été préparées à une concentration active de 1 000 ppm dans une saumure contenant de l'eau, 30 000 ppm de NaCl et 3 000 ppm de $CaCl_2,2H_2O$. Le quotient de filtration (FR) a été mesuré sur des filtres ayant une taille de pore de 3 $\mu$m représentatifs de gisements de faible perméabilité. Les résultats sont présentés sur la figure 10.

Tableau 3 : Polymères testés pour le quotient de filtrabilité

|  | Forme d'ATBS utilisée | Poids moléculaire (en millions Da) | Quotient de filtration |
|---|---|---|---|
| P8 | Cristalline hydratée | 26 | 1,07 |
| P'8 | Non cristalline | 22 | 1,10 |

Nous pouvons observer que, malgré un poids moléculaire plus élevé, le copolymère préparé à partir de la forme cristalline hydratée de l'acide 2-acrylamido-2-méthylpropane sulfonique (P8) présente un FR équivalent à celui du copolymère fabriqué à partir de la forme non cristalline de l'acide 2-acrylamido-2-méthylpropane sulfonique (P'8).

**Revendications**

1. Procédé de récupération assistée du pétrole comprenant les étapes suivantes :

   a) Préparation d'un fluide d'injection comprenant au moins un (co)polymère hydrosoluble préparé au moins à partir d'acide 2-acrylamido-2-méthylpropane sulfonique (ATBS) ou d'au moins un de ses sels, avec de l'eau ou avec de la saumure,
   l'acide 2-acrylamido-2-méthylpropane sulfonique étant une forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta,
   b) Injection du fluide d'injection dans une formation souterraine,
   c) Balayage de la formation souterraine à l'aide du fluide injecté,
   d) Récupération d'un mélange aqueux et hydrocarboné.

2. Procédé selon la revendication 1, *caractérisé* **en ce que** le fluide d'injection comprend entre 10 ppm et 15 000 ppm de (co)polymère hydrosoluble.

3. Procédé selon l'une des revendications précédentes, *caractérisé* **en ce que** le (co)polymère hydrosoluble est préparé au moins à partir d'acide 2-acrylamido-2-méthylpropane sulfonique dont 50% à 100% est sous la forme cristalline hydratée.

4. Procédé selon l'une des revendications précédentes, *caractérisé* **en ce que** le (co)polymère hydrosoluble est un (co)polymère obtenu au moins à partir d'acide 2-acrylamido-2-méthylpropane sulfonique dont 50% à 100% est sous la forme cristalline hydratée et d'au moins un monomère non ionique et/ou au moins un monomère anionique et/ou au moins un monomère cationique et/ou un monomère zwitterionique.

5. Procédé selon la revendication 4, *caractérisé* **en ce que** le monomère non ionique est choisi parmi l'acrylamide, le méthacrylamide, le N-isopropylacrylamide, le N,N-diméthylacrylamide, le N,N-diéthylacrylamide, le N-méthylolacrylamide, la N-vinylformamide, le N-vinyl acétamide, la N-vinylpyridine, la N-vinylpyrrolidone, le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprenol.

6. Procédé selon la revendication 4 ou 5, *caractérisé* **en ce que** le monomère anionique est choisi parmi l'acide acrylique ; l'acide méthacrylique ; l'acide itaconique ; l'acide crotonique ; l'acide maléique ; l'acide fumarique, l'acide acrylamido undécanoïque, l'acide 3-acrylamido 3-méthylbutanoïque, l'anhydride maléique ; l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylméthacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium.

**7.** Procédé selon l'une des revendications précédentes, *caractérisé* **en ce que** le fluide d'injection comprend entre 50 ppm et 10 000 ppm de (co)polymère, avantageusement entre 100 et 5 000 ppm.

**8.** Procédé selon la revendication 1, *caractérisé* **en ce que** le (co)polymère hydrosoluble est un homopolymère préparé à partir de la forme cristalline hydratée de l'acide 2-acrylamido-2-méthylpropane sulfonique.

**Patentansprüche**

**1.** Verfahren zur tertiären Ölgewinnung, umfassend die folgenden Schritte:

a) das Herstellen eines Einspritzfluids, umfassend mindestens ein wasserlösliches (Co-)Polymer, das zumindest aus 2-Acrylamido-2-methylpropansulfonsäure (ATBS) oder mindestens einem ihrer Salze hergestellt ist, mit Wasser oder mit Salzlösung,
wobei die 2-Acrylamido-2-methylpropansulfonsäure eine hydratisierte kristalline Form der 2-Acrylamido-2-methylpropansulfonsäure mit einem Pulver-Röntgenbeugungsdiagramm mit Peaks bei 10,58°, 11,2°, 12,65°, 13,66°, 16,28°, 18,45°, 20°, 20,4° 22,5° 25,5° 25,88°, 26,47°, 28,52°, 30,28°, 30,8°, 34,09°, 38,19°, 40,69°, 41,82°, 43,74°, 46,04° Grad 2-Theta ist,
b) das Einspritzen des Einspritzfluids in eine unterirdische Formation,
c) das Spülen der unterirdischen Formation mit Hilfe des eingespritzten Fluids,
d) das Gewinnen eines wässrigen Kohlenwasserstoffgemisches.

**2.** Verfahren gemäß Anspruch 1, **dadurch gekennzeichnet, dass** das Einspritzfluid zwischen 10 ppm und 15.000 ppm wasserlösliches (Co-)Polymer umfasst.

**3.** Verfahren gemäß einem der vorhergehenden Ansprüche, **dadurch gekennzeichnet, dass** das wasserlösliche (Co-)Polymer zumindest aus 2-Acrylamido-2-methylpropansulfonsäure hergestellt wird, wobei 50% bis 100% davon in hydratisierter kristalliner Form vorliegen.

**4.** Verfahren gemäß einem der vorhergehenden Ansprüche, **dadurch gekennzeichnet, dass** das wasserlösliche (Co-)Polymer ein (Co-)Polymer ist, das zumindest aus 2-Acrylamido-2-methylpropansulfonsäure, wobei 50% bis 100% davon in hydratisierter kristalliner Form vorliegen, und aus mindestens einem nichtionischen Monomer und/oder mindestens einem anionischen Monomer und/oder mindestens einem kationischen Monomer und/oder einem zwitterionischen Monomer gewonnen wird.

**5.** Verfahren gemäß Anspruch 4, **dadurch gekennzeichnet, dass** das nichtionische Monomer aus Acrylamid, Methacrylamid, N-Isopropylacrylamid, N,N-Dimethylacrylamid, N,N-Diethylacrylamid, N-Methylolacrylamid, N-Vinylformamid, N-Vinylacetamid, N-Vinylpyridin, N-Vinylpyrrolidon, N-Vinylimidazol, N-Vinylsuccinimid, Acryloylmorpholin (ACMO), Acryloylchlorid, Glycidylmethacrylat, Glycerylmethacrylat, Diacetonacrylamid und Isoprenol ausgewählt wird.

**6.** Verfahren gemäß Anspruch 4 oder 5, **dadurch gekennzeichnet, dass** das anionische Monomer aus Acrylsäure; Methacrylsäure; Itaconsäure; Crotonsäure; Maleinsäure; Fumarsäure, Acrylamidoundecansäure, 3-Acrylamido-3-methylbuttersäure, Maleinsäureanhydrid; Vinylsulfonsäure, Vinylphosphonsäure, Allylsulfonsäure, Methallylsulfonsäure, 2-Methylidenpropan-1,3-disulfonsäure, 2-Sulfoethylmethacrylat, Sulfopropylmethacrylat, Sulfopropylacrylat, Allylphosphonsäure, Styrolsulfonsäure, 2-Acrylamido-2-methylpropandisulfonsäure; und wasserlöslichen Salzen dieser Monomere wie deren Alkalimetall-, Erdalkalimetall- oder Ammoniumsalzen ausgewählt wird.

**7.** Verfahren gemäß einem der vorhergehenden Ansprüche, **dadurch gekennzeichnet, dass** das Einspritzfluid zwischen 50 ppm und 10.000 ppm (Co-)Polymer, vorteilhafterweise zwischen 100 und 5.000 ppm, umfasst.

**8.** Verfahren gemäß Anspruch 1, **dadurch gekennzeichnet, dass** das wasserlösliche (Co-)Polymer ein aus der hydratisierten kristallinen Form der 2-Acrylamido-2-methylpropansulfonsäure hergestelltes Homopolymer ist.

**Claims**

1. A process for enhanced oil recovery comprising the following steps:

   a. Preparation of an injection fluid comprising at least one water-soluble (co)polymer prepared at least from 2-acrylamido-2-methylpropane sulfonic acid (ATBS) or from at least one of its salts, with water or with brine, where the 2-acrylamido-2-methylpropane sulfonic acid is a hydrated crystalline form of 2-acrylamido-2-methylpropane sulfonic acid having a 2-theta powder X-ray diffraction diagram comprising peaks at 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrees.
   b) Injection of injection fluid into an underground formation,
   c) Flushing of the underground formation using the fluid injected,
   d. Recovery of an aqueous and hydrocarbon mixture.

2. The process according to claim 1, *characterized* **in that** the injection fluid comprises between 10 ppm and 15,000 ppm of water-soluble (co)polymer.

3. The process according to one of the previous claims, *characterized* **in that** the water-soluble (co)polymer is prepared at least from 2-acrylamido-2-methylpropane sulfonic acid of which 50% to 100% is in the hydrated crystalline form.

4. The process according to one of the previous claims, *characterized* **in that** the water-soluble (co)polymer is a (co)polymer obtained at least from 2-acrylamido-2-methylpropane sulfonic acid of which 50% to 100% is in the hydrated crystalline form and of at least one nonionic monomer and/or at least one anionic monomer and/or at least one cationic monomer and/or a zwitterionic monomer.

5. The process according to claim 4, *characterized* **in that** the nonionic monomer is chosen from acrylamide, methacrylamide, N-isopropylacrylamide, N,N-dimethylacrylamide, N,N-diethylacrylamide, N-methylolacrylamide, N-vinylformamide, N-vinyl acetamide, N-vinylpyridine, N-vinylpyrrolidone, N-vinyl imidazole, N-vinyl succinimide, acryloyl morpholine (ACMO), acryloyl chloride, glycidyl methacrylate, glyceryl methacrylate, diacetone acrylamide and isoprenol.

6. The process according to claim 4 or 5, *characterized* **in that** the anionic monomer is chosen from acrylic acid, methacrylic acid, itaconic acid, crotonic acid, maleic acid, fumaric acid, acrylamido undecanoic acid, 3-acrylamido 3-methylbutanoic acid, maleic anhydride, vinylsulfonic acid, vinylphosphonic acid, allylsulfonic acid, methallylsulfonic acid, 2-methylidenepropane-1,3-disulfonic acid, 2-sulfoethylmethacrylate, sulfopropylmethacrylate, sulfopropylacrylate, allylphosphonic acid, styrene sulfonic acid, 2-acrylamido-2-methyl propane disulfonic acid; and water-soluble salts of these monomers like their alkali metal, alkaline earth metal, or ammonium salts.

7. The process according to any of the previous claims, *characterized* **in that** the injection fluid comprises between 50 ppm and 10,000 ppm of (co)polymer, advantageously between 100 and 5,000 ppm.

8. The process according to claim 1, *characterized* **in that** the water-soluble (co)polymer is a homopolymer prepared from the hydrated crystalline form of 2-acrylamido-2-methylpropane sulfonic acid.

| pic | degré 2 theta |
|-----|---------------|
| 1   | 11,43         |
| 2   | 12,97         |
| 3   | 15,26         |
| 4   | 19,19         |
| 5   | 19,61         |
| 6   | 21,86         |
| 7   | 22,56         |
| 8   | 23,06         |
| 9   | 23,80         |
| 10  | 24,52         |
| 11  | 26,16         |
| 12  | 27,24         |

| pic | degré 2 theta |
|-----|---------------|
| 13  | 29,06         |
| 14  | 31,05         |
| 15  | 32,46         |
| 16  | 33,83         |
| 17  | 34,41         |
| 18  | 34,96         |
| 19  | 35,50         |
| 20  | 37,51         |
| 21  | 38,58         |
| 22  | 39,73         |
| 23  | 44,08         |
| 24  | 46,61         |

FIG. 1

| pic | degré 2 theta |
|-----|---------------|
| 1   | 10,58         |
| 2   | 11,2          |
| 3   | 12,65         |
| 4   | 13,66         |
| 5   | 16,28         |
| 6   | 18,45         |
| 7   | 19,05         |
| 8   | 19,99         |
| 9   | 20,4          |
| 10  | 22,5          |
| 11  | 23,15         |
| 12  | 25,5          |
| 13  | 25,88         |

| pic | degré 2 theta |
|-----|---------------|
| 14  | 26,47         |
| 15  | 27,25         |
| 16  | 28,52         |
| 17  | 28,92         |
| 18  | 30,28         |
| 19  | 30,8          |
| 20  | 34,09         |
| 21  | 38,19         |
| 22  | 40,69         |
| 23  | 41,82         |
| 24  | 43,74         |
| 25  | 46,04         |
| 26  | 46,77         |

FIG. 2

18

FIG.3

**FIG. 4**

**FIG. 5**

FIG. 6

FIG. 7

FIG. 8

FIG. 9

**FIG. 10**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8186871 B
- WO 2018020175 A
- WO 2010133258 A

**Littérature non-brevet citée dans la description**

- Oil & gas science and technology. *revue IFP,* 2008, vol. 63 (1), 9-19